(19) Europäisches Patentamt / European Patent Office / Office européen des brevets

(11) **EP 3 603 655 A1**

(12) **EUROPEAN PATENT APPLICATION**
published in accordance with Art. 153(4) EPC

(43) Date of publication:
**05.02.2020 Bulletin 2020/06**

(21) Application number: **18777589.5**

(22) Date of filing: **23.03.2018**

(51) Int Cl.:
*A61K 35/745* (2015.01)     *A23L 33/135* (2016.01)
*A61P 25/00* (2006.01)     *A61P 43/00* (2006.01)
*C12N 1/00* (2006.01)     *C12N 1/20* (2006.01)

(86) International application number:
**PCT/JP2018/011920**

(87) International publication number:
**WO 2018/181069 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.03.2017 JP 2017063471**

(83) **Declaration under Rule 32(1) EPC (expert solution)**

(71) Applicant: **Morinaga Milk Industry Co., Ltd.
Minato-ku
Tokyo 108-8384 (JP)**

(72) Inventors:
• **SAKURAI, Takuma**
  **Zama-shi**
  **Kanagawa 252-8583 (JP)**
• **HASHIKURA, Nanami**
  **Zama-shi**
  **Kanagawa 252-8583 (JP)**
• **SHIMIZU, Kanetada**
  **Zama-shi**
  **Kanagawa 252-8583 (JP)**
• **YAMADA, Akio**
  **Zama-shi**
  **Kanagawa 252-8583 (JP)**

(74) Representative: **Vossius & Partner
Patentanwälte Rechtsanwälte mbB
Siebertstrasse 3
81675 München (DE)**

(54) **COMPOSITION FOR DEGRADING OPIOID PEPTIDE**

(57)     An object of the present invention is to provide an agent for decomposing an opioid peptide or composition for decomposing an opioid peptide that does not restrict dietary habits, does not provide side reactions, even if it is daily used, and thus can be continuously used without any worries. The object is achieved by an agent for decomposing an opioid peptide or composition for decomposing an opioid peptide, pharmaceutical composition for decomposing an opioid peptide, or food or drink composition for decomposing an opioid peptide containing a *Bifidobacterium* bacterium, a culture of the bacterium, and/or a processed cell product of the bacterium as an active ingredient.

**Description**

Technical Field

The present invention relates to an agent for decomposing an opioid peptide or composition for decomposing an opioid peptide, a pharmaceutical composition for decomposing an opioid peptide, and a food or drink composition for decomposing an opioid peptide.

Background Art

Peptides include opioid peptides that bind with an opioid receptor to express various physiological activities. There are also many opioid peptides derived from foods. For example, it is known that an opioid peptide derived from milk and called β-casomorphin is produced from casein, which is a milk protein, in the human alimentary tract. It is known that if such opioid peptides are not decomposed in the alimentary tract, they adversely affect living bodies in various ways. For example, it is known that opioid peptides derived from foods act on cells of the intestinal tract to inhibit absorption of cysteine, and thereby reduce the antioxidation ability (Non-patent document 1).
In order to avoid the influences of such opioid peptides, for example, diets made from such materials that opioid peptides are not produced in the alimentary canal, and so forth have been proposed (Non-patent document 2). Furthermore, for the purpose of assisting digestion of opioid peptides derived from foods, there is disclosed an exogenous opioid peptide decomposition enzyme preparation, which comprises one or more ingredients selected from the group consisting of an enzyme preparation prepared from *Penicillium citrinum,* an enzyme preparation prepared from *Aspergillus oryzae,* and an enzyme preparation prepared from *Aspergillus melleus,* and shows decomposition activities for opioid peptides derived from wheat gluten and opioid peptides derived from casein (Patent document 1). Furthermore, it has also been reported that an enzyme derived from *Lactococcus lactis* subsp. *cremoris* decomposes β-casomorphin, which is an opioid peptide derived from casein (Non-patent document 3).
As for *Bifidobacterium* bacteria, it was disclosed that the *Bifidobacterium longum* IATA-ES1 has an activity for hydrolyzing gliadin, which is a gluten peptide, and therefore it is used for treatment of food allergy (Patent document 2). However, it is not known that *Bifidobacterium* bacteria, cultures of the bacteria, and processed cell products of the bacteria have an enzymatic activity for decomposing opioid peptides.

Prior art references

Patent documents

Patent document 1: International Patent Publication WO2013/047082
Patent document 2: Japanese Patent Laid-open (KOHYO) No. 2011-507540

Non-patent documents

Non-patent document 1: M.S. Trivedi et al., J. Nutr. Biochem., 25(10), pp.1011-1018, 2014
Non-patent document 2: A. Reissmann et al., Functional Foods in Health and Disease, 4(8), pp.349-361, 2014
Non-patent document 3: T-R Yan et al., Biosci. Biotech. Biochem., 56(5), pp.704-707, 1992

Summary of the Invention

Object to be Achieved by the Invention

With diets made from such materials that opioid peptides are not produced in the alimentary canal, a problem of marked restriction of dietary habits is concerned.
An object of the present invention is to provide an agent for decomposing an opioid peptide or composition for decomposing an opioid peptide that does not restrict dietary habits, does not provide side reactions, even if it is daily used, and thus can be continuously used without any worries.

Means for Achieving the Object

The inventor of the present invention conducted various research, as a result, found that *Bifidobacterium* bacteria, cultures of the bacteria, and/or processed cell products of the bacteria have an enzymatic activity similar to that of dipeptidyl peptidase 4 (DPP-4), which has a decomposition activity for opioid peptides, and accomplished the present invention.

Thus, the present invention relates to an agent for decomposing an opioid peptide or composition for decomposing an opioid peptide containing a *Bifidobacterium* bacterium, a culture of the bacterium, and/or a processed cell product of the bacterium as an active ingredient.

In a preferred embodiment of the agent for decomposing an opioid peptide or composition for decomposing an opioid peptide, the opioid peptide is β-casomorphin.

In a preferred embodiment of the agent for decomposing an opioid peptide or composition for decomposing an opioid peptide, the processed cell product of the *Bifidobacterium* bacterium is a water-soluble fraction of a disrupted cell product of the bacterium.

In a preferred embodiment of the agent for decomposing an opioid peptide or composition for decomposing an opioid peptide, the *Bifidobacterium* bacterium is a human-residential bacterium. Furthermore, in a preferred embodiment of the agent for decomposing an opioid peptide or composition for decomposing an opioid peptide, the *Bifidobacterium* bacterium is an infant (baby and little child)-residential bacterium.

Furthermore, in a preferred embodiment of the agent for decomposing an opioid peptide or composition for decomposing an opioid peptide, the *Bifidobacterium* bacterium is one or more selected from the group consisting of *Bifidobacterium longum* subsp. *longum* ATCC 15707, *Bifidobacterium longum* subsp. *longum* ATCC BAA-999 or *Bifidobacterium longum* subsp. *longum* BB536 (NITE BP-02621), *Bifidobacterium longum* subsp. *infantis* ATCC 15697, *Bifidobacterium longum* subsp. *infantis* BCCM LMG23728 or *Bifidobacterium longum* subsp. *infantis* M-63 (NITE BP-02623), *Bifidobacterium breve* ATCC 15700, *Bifidobacterium breve* FERM BP-11175, *Bifidobacterium breve* BCCM LMG23729 or *Bifidobacterium breve* M-16V (NITE BP-02622), *Bifidobacterium bifidum* ATCC 29521, *Bifidobacterium adolescentis* ATCC 15703, *Bifidobacterium angulatum* ATCC 27535, *Bifidobacterium dentium* DSM20436, *Bifidobacterium psudocatenulatum* ATCC 27919, *Bifidobacterium animalis* subsp. *lactis* DSM10140, *Bifidobacterium animalis* subsp. *animalis* ATCC 25527, *Bifidobacterium pseudolongum* subsp. *globosum* JCM5820, *Bifidobacterium pseudolongum* subsp. *pseudolongm* ATCC 25526, and *Bifidobacterium thermophilum* ATCC 25525.

The present invention also relates to a pharmaceutical composition for decomposing an opioid peptide containing a *Bifidobacterium* bacterium, a culture of the bacterium, and/or a processed cell product of the bacterium as an active ingredient.

According to a preferred embodiment of the pharmaceutical composition, it is used for prevention and/or treatment of autism, Asperger's syndrome, Rett's disorder, childhood disintegrative disorder, or sleep apnea.

The present invention also relates to a food or drink composition for decomposing an opioid peptide containing a *Bifidobacterium* bacterium, a culture of the bacterium, and/or a processed cell product of the bacterium as an active ingredient.

The present invention also relates to use of a *Bifidobacterium* bacterium, a culture of the bacterium, and/or a processed cell product of the bacterium in manufacture of a composition for decomposing an opioid peptide.

The present invention also relates to a *Bifidobacterium* bacterium, a culture of the bacterium, and/or a processed cell product of the bacterium, which is used for decomposing an opioid peptide.

The present invention also relates to use of a *Bifidobacterium* bacterium, a culture of the bacterium, and/or a processed cell product of the bacterium for decomposing an opioid peptide.

The present invention also relates to a method for decomposing an opioid peptide, which comprises the step of administering a *Bifidobacterium* bacterium, a culture of the bacterium, and/or a processed cell product of the bacterium to a mammal.

Effect of the Invention

Since the agent for decomposing an opioid peptide or composition for decomposing an opioid peptide, pharmaceutical composition for decomposing an opioid peptide, and food or drink composition for decomposing an opioid peptide of the present invention containing a *Bifidobacterium* bacterium, a culture of the bacterium, and/or a processed cell product of the bacterium as an active ingredient uses a *Bifidobacterium* bacterium, a culture of the bacterium, and/or a processed cell product of the bacterium, which have been used as an ingredient of oral compositions for many years, as an active ingredient, they can be administered to patients suffering from various diseases without any worries. Since the *Bifidobacterium* bacteria exist also in the intestines of humans and animals, they are expected to hardly provide side reactions even if they are continuously administered for a long period of time.

Therefore, according to the present invention, there can be provided an agent for decomposing an opioid peptide or composition for decomposing an opioid peptide, pharmaceutical composition for decomposing an opioid peptide, and food or drink composition for decomposing an opioid peptide that does not restrict dietary habits, does not provide side reactions, even if they are daily used, and thus can be continuously used without any worries. Therefore, absorption from the alimentary canal of opioid peptides mainly derived from foods and not decomposed can be suppressed by use of the agent for decomposing an opioid peptide or composition for decomposing an opioid peptide, or food or drink composition for decomposing an opioid peptide.

Modes for Carrying out the Invention

Hereafter, preferred embodiments of the present invention will be explained. However, the present invention is not limited to the following embodiments, and can be freely embodied within the scope of the present invention. The percentages mentioned in this description are used on mass-basis unless especially indicated.

<Agent for decomposing opioid peptide or composition for decomposing opioid peptide>

The agent for decomposing an opioid peptide of the present invention is for decomposing an opioid peptide mainly derived from a food, and contains a *Bifidobacterium* bacterium, a culture of the bacterium and/or a processed cell product of the bacterium as an active ingredient. The agent for decomposing an opioid peptide of the present invention may contain other ingredients so long as it contains a *Bifidobacterium* bacterium, a culture of the bacterium, and/or a processed cell product of the bacterium as an active ingredient. That is, the agent for decomposing an opioid peptide of the present invention may be a composition for decomposing an opioid peptide. Therefore, another aspect of the present invention is a composition for decomposing an opioid peptide containing a *Bifidobacterium* bacterium, a culture of the bacterium, and/or a processed cell product of the bacterium as an active ingredient.

The content of the *Bifidobacterium* bacterium in the composition is preferably $1 \times 10^6$ to $1 \times 10^{12}$ CFU/g or $1 \times 10^6$ to $1 \times 10^{12}$ CFU/mL, more preferably $1 \times 10^7$ to $1 \times 10^{11}$ CFU/g or $1 \times 10^7$ to $1 \times 10^{11}$ CFU/mL, further preferably $1 \times 10^8$ to $1 \times 10^{10}$ CFU/g or $1 \times 10^8$ to $1 \times 10^{10}$ CFU/mL. When the bacterium consists of dead cells, the unit shall be number of cells (cells) instead of CFU.

The *Bifidobacterium* bacterium, a culture of the bacterium, and a processed cell product of the bacterium used for the present invention have the same enzyme activity as that of dipeptidyl peptidase 4 (DPP-4), which is a kind of dipeptidyl aminopeptidase.

DPP-4 is an exopeptidase having an activity of cleaving a dipeptide from the end of a protein or peptide on the amino terminus side, and it is known as an enzyme having an activity for decomposing casomorphins, which are opioid peptides (G. Puschel et al., Eur. J. Biochem., 126 (2), pp.359-365, 1982).

The "DPP-4 activity" referred to in this description means the same enzymatic activity as that of DPP-4 or an enzymatic activity similar to the same, specifically, an activity for decomposing a substrate specific to DPP-4. Examples of the substrate specific to DPP-4 include a protein and peptide having a proline or alanine residue as the second amino acid residue from the amino terminus.

Another aspect of the present invention is a method for producing a composition for decomposing an opioid peptide, which comprises the step of adding the *Bifidobacterium* bacterium to a raw material of the composition. The production method may be a method for producing a food or drink composition, which comprises the step of adding the *Bifidobacterium* bacterium to a food or drink raw material. The production method may also be a method for producing a pharmaceutical composition, which comprises the step of adding a *Bifidobacterium* bacterium to a raw material of the pharmaceutical composition (e.g., base, etc.).

The *Bifidobacterium* bacterium used in the method for producing a composition for decomposing an opioid peptide may consist of live cells or dead cells, or both live cells and dead cells. It may also consist of a culture of the bacterium, or a processed cell product of the bacterium. In the method for producing a composition for decomposing an opioid peptide, the *Bifidobacterium* bacterium may be added to a raw material of the composition after it is cultured, and concentrated or lyophilized, or a *Bifidobacterium* bacterium may be cultured after adding it to a raw material of the composition.

(1) *Bifidobacterium* bacterium

As the *Bifidobacterium* bacterium used for the present invention, a known *Bifidobacterium* bacterium can be used so long as the effect of the present invention is not degraded. Examples include, for example, *Bifidobacterium longum* subsp. *longum, Bifidobacterium longum* subsp. *infantis, Bifidobacterium breve, Bifidobacterium bifidum, Bifidobacterium adolescentis, Bifidobacterium angulatum, Bifidobacterium dentium, Bifidobacterium pseudocatenulatum, Bifidobacterium animalis* subsp. *lactis, Bifidobacterium animalis* subsp. *animalis, Bifidobacterium pseudolongum* subsp. *globosum, Bifidobacterium pseudolongum* subsp. *pseudolongum, Bifidobacterium thermophilum,* and so forth. *Bifido-*

*bacterium longum* subsp. *longum* may be referred to simply as *Bifidobacterium longum*. *Bifidobacterium longum* subsp. *infantis* may be referred to simply as *Bifidobacterium infantis*.

Specific examples include *Bifidobacterium longum* subsp. *longum* ATCC 15707, *Bifidobacterium longum* subsp. *longum* ATCC BAA-999 or *Bifidobacterium longum* subsp. *longum* BB536 (NITE BP-02621), *Bifidobacterium longum* subsp. *infantis* ATCC 15697, *Bifidobacterium longum* subsp. *infantis* BCCM LMG23728 or *Bifidobacterium longum* subsp. *infantis* M-63 (NITE BP-02623), *Bifidobacterium breve* ATCC 15700, *Bifidobacterium breve* FERM BP-11175, *Bifidobacterium breve* BCCM LMG23729 or *Bifidobacterium breve* M-16V (NITE BP-02622), *Bifidobacterium bifidum* ATCC 29521, *Bifidobacterium adolescentis* ATCC 15703, *Bifidobacterium angulatum* ATCC 27535, *Bifidobacterium dentium* DSM20436, *Bifidobacterium Bifidobacterium pseudocatenulatum* ATCC 27919, *Bifidobacterium animalis* subsp. *lactis* DSM10140, *Bifidobacterium animalis* subsp. *animalis* ATCC 25527, *Bifidobacterium pseudolongum* subsp. *globosum* JCM5820, *Bifidobacterium pseudolongum* subsp. *pseudolongum* ATCC 25526, *Bifidobacterium thermophilum* ATCC 25525, and so forth. As the *Bifidobacterium* bacterium, only one kind of bacterium may be used, or arbitrary two or more kinds of bacteria may be used.

The bacteria mentioned with ATCC numbers can be obtained from American Type Culture Collection (12301 Parklawn Drive, Rockville, Maryland 20852, United States of America).

The bacteria mentioned with BCCM numbers can be obtained from the preservation organization of Belgium, Belgian Coordinated Collections of Microorganisms (Rue de la Science 8, Wetenschapsstraat, B-1000 Brussels, Belgium).

The bacterium given the accession number of FERM BP-11175 was deposited at the independent administrative agency, National Institute of Advanced Industrial Science and Technology, International Patent Organism Depository (presently the independent administrative agency, National Institute of Technology and Evaluation, International Patent Organism Depository, #120, 2-5-8 Kazusakamatari, Kisarazu-shi, Chiba-ken, 292-0818, Japan) on August 25, 2009 as an international deposit according to the provisions of the Budapest Treaty.

The bacteria mentioned with DSM numbers can be obtained from Deutsche Sammlung von Mikroorganismen und Zellkulturen GmbH (Inhoffenstrabetae 7B, 38124 Braunschweig, Germany).

The bacteria mentioned with JCM numbers can be obtained from Japan Collection of Microorganisms (the national research and development agency, Institute of Physical and Chemical Research (RIKEN), BioResource Research Center, Microbe Division (3-1-1, Koyadai, Tsukuba-shi, Ibaraki-ken, Japan, 305-0074).

*Bifidobacterium longum* subsp. *longum* ATCC BAA-999 is the same as *Bifidobacterium longum* subsp. *longum* BB536 (NITE BP-02621), and either one of them can be used for a preferred embodiment of the present invention. *Bifidobacterium longum* subsp. *longum* BB536 (NITE BP-02621) was deposited at the independent administrative agency, National Institute of Technology and Evaluation, NITE Patent Microorganisms Depositary (#122, 2-5-8 Kazusakamatari, Kisarazu-shi, Chiba-ken, 292-0818, Japan) on January 26, 2018 as an international deposit according to the provisions of the Budapest Treaty, and given the accession number NITE BP-02621.

*Bifidobacterium longum* subsp. *infantis* BCCM LMG23728 is the same as *Bifidobacterium longum* subsp. *infantis* M-63 (NITE BP-02623), and either one of them can be used for a preferred embodiment of the present invention. *Bifidobacterium longum* subsp. *infantis* M-63 (NITE BP-02623) was deposited at the independent administrative agency, National Institute of Technology and Evaluation, NITE Patent Microorganisms Depositary (#122, 2-5-8 Kazusakamatari, Kisarazu-shi, Chiba-ken, 292-0818, Japan) on January 26, 2018 as an international deposit according to the provisions of the Budapest Treaty, and given the accession number NITE BP-02623.

*Bifidobacterium breve* BCCM LMG23729 is the same as *Bifidobacterium breve* M-16V (NITE BP-02622), and either one of them can be used for a preferred embodiment of the present invention. *Bifidobacterium breve* M-16V (NITE BP-02622) was deposited at the independent administrative agency, National Institute of Technology and Evaluation, NITE Patent Microorganisms Depositary (#122, 2-5-8 Kazusakamatari, Kisarazu-shi, Chiba-ken, 292-0818, Japan) on January 26, 2018 as an international deposit according to the provisions of the Budapest Treaty, and given the accession number NITE BP-02622.

The *Bifidobacterium* bacterium used for the present invention is not limited to the aforementioned deposited strains, but may be a bacterium substantially equivalent to any one of the deposited strains. Such a bacterium substantially equivalent to any one of the deposited strains refers to a bacterium of the same genus or species as those of the corresponding deposited strain that can decompose an opioid peptide in an subject when the bacterium, a culture of the bacterium, and/or a processed cell product of the bacterium is ingested by or administered to the subject, has a 16S rRNA gene nucleotide sequence showing a homology of 98% or higher, preferably 99% or higher, more preferably 100%, to the nucleotide sequence of 16S rRNA gene of the corresponding deposited strain, and preferably has the same bacteriological characteristics as those of the corresponding deposited strain in addition to the aforementioned homology. The *Bifidobacterium* bacterium used for the present invention may be a strain bred from any one of the deposited strains or a bacterium substantially equivalent to any one of deposited strains by mutation treatment, gene recombination, selection of a spontaneously mutated strain, or the like, so long as the effect of the present invention is not degraded.

Among these, a human-residential *Bifidobacterium* bacterium is preferably used for the present invention.

Although *Bifidobacterium* bacteria inhabit insects and small animals in addition to humans, the habitat (host) is limited for each species. And *Bifidobacterium* bacteria that mainly inhabit humans are called human-residential bifidobacteria (HRB), and the other *Bifidobacterium* bacteria are called non-HRB. Examples of HRB include *Bifidobacterium longum, Bifidobacterium longum* subsp. *infantis, Bifidobacterium breve, Bifidobacterium bifidum, Bifidobacterium adolescentis, Bifidobacterium angulatum, Bifidobacterium dentium, Bifidobacterium pseudocatenulatum,* and so forth.

For the present invention, among HRB, infant (baby and little child)-residential bifidobacteria are more preferred. Examples of HRB as infant-residential bifidobacteria include *Bifidobacterium longum, Bifidobacterium longum* subsp. *infantis, Bifidobacterium breve, Bifidobacterium bifidum,* and so forth.

The *Bifidobacterium* bacterium used for the present invention may be a variant strain of any one of the aforementioned *Bifidobacterium* bacteria, so long as it has an opioid peptide decomposition effect equivalent to or higher than that of any one of the aforementioned *Bifidobacterium* bacteria. Whether a certain variant strain has an "opioid peptide decomposition effect equivalent to or higher than that of any one of the aforementioned *Bifidobacterium* bacteria" can be confirmed by, for example, measuring the DPP-4 activity of the *Bifidobacterium* bacterium, or measuring the consumption ratio (decomposition rate) of β-casomorphin, which is an opioid peptide, as described later.

Such a variant strain may be obtained by non-artificial introduction of a mutation into any one of the aforementioned *Bifidobacterium* bacteria. It may also be constructed by introducing a mutation into any one of the aforementioned bacteria by a treatment using a mutagen such as UV, or constructed by introducing a mutation into any one of the aforementioned bacteria by any of various gene manipulation methods.

In the present invention, the DPP-4 activity can be confirmed by measuring fluorescence intensity of a culture obtained by incubating a *Bifidobacterium* bacterium, a culture of the bacterium and/or a processed cell product of the bacterium as a mixture containing a fluorescent substrate specific to DPP-4. Namely, concentration of a fluorescent substance originating from the fluorescent substrate in the culture can be calculated from the fluorescence intensity of the culture using a calibration curve representing the relationship between the fluorescence intensity and the concentration of the fluorescent substance, and the enzymatic activity (DPP-4 activity) of the *Bifidobacterium* bacterium, culture of the bacterium and/or processed cell product of the bacterium can be determined in terms of enzyme unit (U), with which the activity for producing 1 nmol of the fluorescent substance in 1 minute is represented as 1 U.

For example, cells are separated from a culture of the *Bifidobacterium* bacterium, and suspended in a PBS solution to prepare a suspension, the suspension is diluted to adjust the turbidity (OD600) thereof to be 0.1, then H-Gly-Pro-AMC·HBr (produced by BACHEM), which is a fluorescent substrate specific to DPP-4, is added, anaerobic culture is performed at 37°C for 60 minutes, then after completion of the culture, fluorescence intensity of the culture is measured with an excitation wavelength of 380 nm and measurement wavelength of 460 nm using a fluorometry apparatus such as Microplate Reader SH-9000 (produced by Corona Electric), and the enzymatic activity (DPP-4 activity) can be determined from the measured fluorescence intensity of the culture.

According to the present invention, if the DPP-4 activity is preferably 0.5 mU or higher, more preferably 0.7 mU or higher, further preferably 1.0 mU or higher, the opioid peptide decomposition effect of the present invention can be favorably obtained.

In the present invention, consumption ratio (decomposition ratio) of β-casomorphin can be calculated in accordance with the following equation from β-casomorphin concentration in a culture obtained by culturing a *Bifidobacterium* bacterium as a mixture with β-casomorphin. [Consumption ratio (%) of β-casomorphin] = 100 - [(β-casomorphin concentration in culture after culture)/(β-casomorphin concentration in culture before culture)] x 100

Specifically, it can be confirmed as described in Test Example 3 described later.

According to the present invention, if the consumption ratio (decomposition ratio) of β-casomorphin is preferably 40% or higher, more preferably 50% or higher, further preferably 60% or higher, the opioid peptide decomposition effect of the present invention can be favorably obtained.

A *Bifidobacterium* bacterium or a culture of the *Bifidobacterium* bacterium used for the present invention can be easily obtained by culturing the *Bifidobacterium* bacterium in a conventional manner. The culture method is not particularly limited so long as the *Bifidobacterium* bacterium can proliferate, and the culture can be performed under appropriate conditions determined according to the characteristics of the bacterium. For example, the culture temperature may be 25 to 50°C, preferably 35 to 42°C. The culture is preferably performed under anaerobic conditions. For example, the culture can be performed with supply of anaerobic gas such as carbon dioxide. The culture may also be performed under microaerobic conditions such as those obtainable with stationary liquid culture, or the like.

The medium for culturing a *Bifidobacterium* bacterium used for the present invention is not particularly limited, and a medium usually used for culture of *Bifidobacterium* bacteria can be used. That is, as the carbon source, for example, saccharides such as glucose, galactose, lactose, arabinose, mannose, sucrose, starch, starch hydrolysates, and blackstrap molasses can be used according to the assimilation property. As the nitrogen source, for example, ammonia, ammonium salts such as ammonium sulfate, ammonium chloride, and ammonium nitrate, and nitrates can be used. As inorganic salts, for example, sodium chloride, potassium chloride, potassium phosphate, magnesium sulfate, calcium chloride, calcium nitrate, manganese chloride, ferrous sulfate, and so forth can be used. Organic components such as peptone, soybean flour, degreased soybean meal, meat extract, and yeast extract may also be used.

As explained above, the *Bifidobacterium* bacterium used for the present invention can be used in the form of the bacterium per se, a culture of the bacterium, or a processed cell product of the bacterium. That is, the bacterium per se may be used, a culture obtained after culture of the bacterium may be used as it is, the obtained culture may be used after dilution or concentration, or cells collected from the culture may be used. The *Bifidobacterium* bacterium used in the present invention may consist of live cells or dead cells, or both live cells and dead cells.

Examples of the processed cell product include, for example, immobilized cells immobilized with acrylamide, carragheenan, or the like, disrupted cell product in which cell walls and cell membranes of the cells are partially or completely disrupted in a conventional manner such as by ultrasonication or homogenizer treatment, and so forth.

The disrupted cell product may be a total fraction obtained after the disruption or a partial fraction thereof, a centrifugation supernatant of the disrupted product, a fraction obtained by partial purification of the supernatant by ammonium sulfate treatment or the like, or a concentrate of the supernatant.

Specific examples of the form of the *Bifidobacterium* bacterium used for the present invention include, for example, a cell suspension thereof, a water-soluble fraction of a disrupted cell product of the cells (bacteria), a precipitate re-suspension thereof, and so forth, and among these, a water-soluble fraction of a disrupted cell product of the cells (bacteria) is preferred, because of high opioid peptide decomposition activity thereof.

The "cell suspension" referred to in this description is a liquid in which cells obtained by centrifugation or the like of a culture of a *Bifidobacterium* bacterium used for the present invention are suspended, and it is preferably a liquid obtained by the procedure described in Test Example 2, (2) mentioned later in the section of Examples.

The "water-soluble fraction" referred to in this description is a supernatant obtained by centrifugation or the like of the aforementioned disrupted cell product, preferably a supernatant finally obtained by the procedure described in Test Example 2, (2) described later in the section of Examples. The water-soluble fraction is more preferably a water-soluble fraction appropriately purified or concentrated in a conventional manner.

The "precipitate re-suspension" referred to in this description is a liquid obtained by suspending precipitates obtained by centrifugation or the like of the aforementioned disrupted cell product, preferably a liquid obtained by suspending precipitates finally obtained by the procedure described in Test Example 2, (2) described later in the section of Examples.

(2) Opioid peptide

Opioid peptides are peptides that bind with an opioid receptor to express various physiological activities. The opioid peptides include endogenous opioid peptides synthesized in the living bodies, and exogenous opioid peptides. The opioid peptide referred to in the present invention is preferably an exogenous opioid peptide, more preferably an opioid peptide derived from food. Examples of the opioid peptide derived from food include β-casomorphin, gliadorphin, and so forth.

<Pharmaceutical composition>

The agent for decomposing an opioid peptide or composition for decomposing an opioid peptide of the present invention can be used as a pharmaceutical composition. It is known that if an opioid peptide is not decomposed and absorbed from the alimentary tract into the bodies, it affects conditions of autism, Asperger's syndrome, Rett's disorder, childhood disintegrative disorder, and so forth (Patent document 1). For example, it has been reported that a child with autism showing a higher urinary concentration of β-casomorphin, which is an opioid peptide, shows a correspondingly higher childhood autism rating scale (CARS) value (O. Sokolov et al., Peptides, 56, pp.68-71, 2014). It has also been reported that the blood β-casomorphin concentration of an infant developing an apparent life-threatening event (ALTE), such as apnea, is higher than that of a healthy infant, and the activity of DPP-4, which is an enzyme that decomposes β-casomorphin, of such an infant is lower than that of a healthy infant (J. Wasilewska et al., Nuropeptides, 45, pp.189-195, 2011).

Therefore, the pharmaceutical composition of the present invention can be used for prevention and/or treatment of a disease in which an opioid peptide is involved. Examples of such a disease in which an opioid peptide is involved include autism, Asperger's syndrome, Rett's disorder, childhood disintegrative disorder, sleep apnea, and so forth.

Since the pharmaceutical composition of the present invention uses a *Bifidobacterium* bacterium, a culture of the bacterium, and/or a processed cell product of the bacterium used for many years as an ingredient of oral compositions as an active ingredient, it can be administered to patients of various diseases without anxiety. Since the *Bifidobacterium* bacteria exist also in the intestines of humans and animals, they are expected to hardly provide side reactions even when they are continuously administered for a long period of time. Furthermore, since the agent for decomposing an opioid peptide or composition for decomposing an opioid peptide of the present invention uses a *Bifidobacterium* bacterium, a culture of the bacterium and/or a processed cell product of the bacterium as an active ingredient, it can also be safely administered to infants and children. Therefore, the agent for decomposing an opioid peptide or composition for decomposing an opioid peptide of the present invention is suitable for prevention and/or treatment of a disease of an infant or child.

When the agent for decomposing an opioid peptide or composition for decomposing an opioid peptide of the present invention is used as a pharmaceutical composition, the pharmaceutical composition may be orally administered or parenterally administered, and it can be appropriately prepared in a desired dosage form depending on the administration method. For example, in the case of oral administration, it can be prepared in the form of solid preparation such as powder, granule, tablet, and capsule, liquid agent such as solution, syrup, suspension, and emulsion, or the like. In the case of parenteral administration, it can be prepared in the form of suppository, ointment, or the like.

When the pharmaceutical composition is prepared, such ingredients as an excipient, pH adjustor, colorant, and corrigent, which are usually used for preparation of pharmaceutical compositions, can be used in addition to the agent for decomposing an opioid peptide or composition for decomposing an opioid peptide of the present invention. So long as the effect of the present invention is not degraded, the agent for decomposing an opioid peptide or composition for decomposing an opioid peptide of the present invention may be used together with a known ingredient or ingredient to be found in future having an effect for preventing and/or treating a disease in which an opioid peptide is involved.

The pharmaceutical composition can be prepared by a known method appropriately chosen depending on the dosage form. The pharmaceutical composition may also be prepared by adding a carrier for pharmaceutical compositions.

Although intake or dose of the pharmaceutical composition of the present invention can be appropriately selected depending on the dosage form thereof, the daily intake or dose per 1 kg of body weight of the *Bifidobacterium* bacterium is, for example, preferably $1 \times 10^6$ to $1 \times 10^{12}$ CFU/kg/day, more preferably $1 \times 10^7$ to $1 \times 10^{11}$ CFU/kg/day, further preferably $1 \times 10^8$ to $1 \times 10^{10}$ CFU/kg/day. CFU contained in the units mentioned above is an abbreviation of colony forming unit. When the bacterium cells consist of dead cells, the amount thereof can be represented in terms of number of cells (cells) instead of CFU.

When a culture of a *Bifidobacterium* bacterium or a processed cell product of the bacterium is used, intake or dose thereof is preferably such an intake or dose that the intake or dose of the *Bifidobacterium* bacterium is within any of the ranges of the intake or dose mentioned above.

Although the content of the *Bifidobacterium* bacterium in the pharmaceutical composition of the present invention can be appropriately selected according to the intake or dose mentioned above, it is, for example, $1 \times 10^6$ to $1 \times 10^{12}$ CFU/g or $1 \times 10^6$ to $1 \times 10^{12}$ CFU/mL, preferably $1 \times 10^7$ to $1 \times 10^{11}$ CFU/g or $1 \times 10^7$ to $1 \times 10^{11}$ CFU/mL, more preferably $1 \times 10^8$ to $1 \times 10^{10}$ CFU/g or $1 \times 10^8$ to $1 \times 10^{10}$ CFU/mL. When the bacterium cells consist of dead cells, the amount thereof can be represented in terms of number of cells (cells) instead of CFU.

When a culture of a *Bifidobacterium* bacterium, or a processed cell product of the bacterium is used, the content thereof is preferably such that the content of the *Bifidobacterium* bacterium is within any of the ranges of *Bifidobacterium* bacterium content mentioned above.

As the aforementioned carrier for pharmaceutical composition, various organic or inorganic carriers can be used depending on the dosage form. In the case of solid preparation, examples of the carrier include, for example, an excipient, binder, disintegrating agent, lubricant, stabilizer, corrigent, and so forth.

Examples of the excipient include, for example, sugar and derivatives thereof such as lactose, sucrose, glucose, mannitol, and sorbitol; starch and derivatives thereof such as corn starch, potato starch, pregelatinized starch, dextrin, and carboxymethyl starch; cellulose and derivatives thereof such as crystalline cellulose, hydroxypropylcellulose, hydroxypropylmethylcellulose, carboxymethylcellulose, and carboxymethylcellulose calcium; gum arabic; dextran; pullulan; silicate and derivatives thereof such as light silicic anhydride, synthetic aluminum silicate and magnesium metasilicate aluminate; phosphate and derivatives thereof such as calcium phosphate; carbonate derivatives such as calcium carbonate; sulfate derivatives such as calcium sulfate, and so forth.

Examples of the binder include, in addition to the aforementioned excipients, for example, gelatin; polyvinylpyrrolidone; Macrogol, and so forth.

Examples of the disintegrating agent include, in addition to the aforementioned excipients, for example, chemically modified starch or cellulose derivatives such as croscarmellose sodium, and carboxymethylstarch sodium, crosslinked polyvinylpyrrolidone, and so forth.

Examples of the lubricant include, for example, talc; stearic acid; stearic acid metal salts such as calcium stearate and magnesium stearate; colloidal silica; waxes such as peegum and spermaceti; boric acid; glycols; carboxylic acids such as fumaric acid and adipic acid; carboxylic acid sodium salts such as sodium benzoate; sulfates such as sodium sulfate; leucine; laurylsulfates such as sodium laurylsulfate and magnesium laurylsulfate; silicic acids such as silicic anhydride and silicic acid hydrate; starch derivatives, and so forth.

Examples of the stabilizer include, for example, p-hydroxybenzoic acid esters such as methylparaben and propylparaben; alcohols such as chlorobutanol, benzyl alcohol and phenylethyl alcohol; benzalkonium chloride; acetic anhydride; sorbic acid, and so forth.

Examples of the corrigent include, for example, sweetener, acidulant, perfume, and so forth.

Examples of the carrier used for liquids for oral administration include solvents such as water, corrigents, and so forth.

<Food or drink composition>

The agent for decomposing an opioid peptide or composition for decomposing an opioid peptide of the present invention can also be used as a food or drink composition. The food or drink composition of the present invention may be produced by adding a *Bifidobacterium* bacterium, a culture of the bacterium, and/or a processed cell product of the bacterium to a known food or drink, or may be prepared as a novel food or drink by adding a *Bifidobacterium* bacterium, a culture of the bacterium and/or a processed cell product of the bacterium to a raw material of a food or drink. The food or drink composition of the present invention may also be produced by a production method comprising the step of, after a *Bifidobacterium* bacterium is added to a raw material of a food or drink, performing culture of the bacterium.

When the food or drink composition containing a *Bifidobacterium* bacterium is orally ingested, the *Bifidobacterium* bacterium that proliferates in the body (in the intestines) decompose opioid peptides, and therefore it can suppress absorption of undecomposed opioid peptides from the alimentary canal into the body.

The form of the food or drink composition of the present invention is not particularly limited, and it may be in the form of a liquid, paste, solid, powder, or the like. Examples include tablet confectioneries, liquid diets, feeds (including feeds for pets), and so forth, as well as, for example, flour products, ready-to-eat foods; processed agricultural products, processed marine products, processed livestock products, milks and dairy products, oils and fats, basic seasonings, complex seasonings and foods, frozen foods, confectioneries, beverages, other commercial foods, and so forth. So long as the effect of the present invention is not degraded, a known ingredient or ingredient to be found in the future that has a probiotics effect or assists a probiotics effect may be used in the food or drink composition of the present invention. The food or drink composition of the present invention may contain, for example, various proteins such as whey proteins, casein proteins, soybean proteins, and green pea proteins (pea proteins), or a mixture or decomposition product of these; amino acids such as leucine, valine, isoleucine, and glutamine; vitamins such as vitamin B6 and vitamin C; creatine; citric acid; fish oil; and oligosaccharides such as isomaltooligosaccharides, galactooligosaccharides, xylooligosaccharides, soybean oligosaccharides, fructooligosaccharides, and lactulose.

The food or drink composition of the present invention can also be provided and sold as a food or drink with an indication of use thereof (including health use) such as prevention of a disease in which an opioid peptide is involved, reduction of risk of such a disease, amelioration of conditions of such a disease, and/or treatment of such a disease.

The aforementioned term "indication" means all actions for informing consumers the aforementioned use, and any indications reminding or analogizing the aforementioned use fall within the scope of the "indication" referred to in the present invention regardless of purpose and content of the indication, objective article or medium on which the indication is used, and so forth.

The indication is preferably made with an expression that allows consumers to directly recognize the aforementioned use. Specific examples include actions of assigning or delivering commodities including the food or drink on which the aforementioned use is indicated or such commodities having packages on which the aforementioned use is indicated, displaying or importing such commodities, displaying or distributing advertisements, price lists or business papers relating to such commodities on which the aforementioned use is indicated, providing information including those as contents with indicating the aforementioned use by an electromagnetic method (Internet etc.) for the purpose of assigning or delivering such commodities, and so forth.

The indication is preferably an indication approved by the administration etc. (for example, an indication in a form based on an approval, which is qualified on the basis of any of various legal systems provided by the administration). Indications of such contents on packages, containers, catalogues, pamphlets, advertisement materials used at the sales spots such as POPs, and other papers are especially preferred.

Examples of the "indication" include, for example, indications as health food, functional food, enteral nutritive food, food for special dietary uses, food with health claims, food for specified health uses, food with nutrient function claims, food with function claims, quasi-drug, and so forth. Among these, indications approved by the Consumer Affairs Agency, for example, indications approved on the basis of the systems of food for specified health uses, food with nutrient function claims, food with function claims, and similar systems can be especially mentioned. Specific examples include indications as food for specified health uses, indications as food for specified health uses with qualified health claims, indications of influence on body structures and functions, indications of reduction of disease risk claims, indications of functional claims based on scientific evidence, and so forth, and more precisely, typical examples include the indications as food for specified health uses (especially indications of use for health) defined in the Cabinet Office Ordinance concerning approval of indications of special dietary uses provided by the Health Promotion Law, and others (Cabinet Office Ordinance No. 57, August 31, 2009), and similar indications.

Intake of the food or drink composition of the present invention can be appropriately selected. For example, the daily intake of the *Bifidobacterium* bacterium per 1 kg of body weight is preferably $1 \times 10^6$ to $1 \times 10^{12}$ CFU/kg/day,

more preferably $1 \times 10^7$ to $1 \times 10^{11}$ CFU/kg/day, further preferably $1 \times 10^8$ to $1 \times 10^{10}$ CFU/kg/day. When the bacterium consists of dead cells, the unit CFU should be replaced by number of cells (cells).

When a culture of a *Bifidobacterium* bacterium, or a processed cell product of the bacterium is used, intake thereof is preferably such an intake that the intake of the *Bifidobacterium* bacterium is within any of the ranges of intake mentioned above.

Although the content of a *Bifidobacterium* bacterium in the food or drink composition of the present invention can be appropriately selected according to the intake mentioned above, it may be, for example, $1 \times 10^6$ to $1 \times 10^{12}$ CFU/g or $1 \times 10^6$ to $1 \times 10^{12}$ CFU/mL, preferably $1 \times 10^7$ to $1 \times 10^{11}$ CFU/g or $1 \times 10^7$ to $1 \times 10^{11}$ CFU/mL, more preferably $1 \times 10^8$ to $1 \times 10^{10}$ CFU/g or $1 \times 10^8$ to $1 \times 10^{10}$ CFU/mL. When the bacterium consists of dead cells, CFU should be replaced with number of cells (cells).

When a culture of a *Bifidobacterium* bacterium, or a processed cell product of the bacterium is used, the content thereof is preferably such that the content of the *Bifidobacterium* bacterium is within any of the ranges of content mentioned above.

The food or drink composition for decomposing an opioid peptide of the present invention can be used as a food or drink composition for humans or animals. The food or drink composition for decomposing an opioid peptide of the present invention is effective for prevention and/or treatment of a disease in which an opioid peptide is involved, such as autism, Asperger's syndrome, Rett's disorder, childhood disintegrative disorder, and sleep apnea. The food or drink composition for decomposing an opioid peptide of the present invention contains a *Bifidobacterium* bacterium, a culture of the bacterium and/or a processed cell product of the bacterium as an active ingredient, and therefore it can be safely administered even to infants or children. Accordingly, the food or drink composition for decomposing an opioid peptide of the present invention is suitable also for prevention and/or treatment of a disease in infants or children.

The other aspects of the present invention are as follows. In the following descriptions, mammal may be human, bovine, sheep, goat, pig, dog, cat, horse, or the like.

[1] Use of a *Bifidobacterium* bacterium, a culture of the bacterium, and/or a processed cell product of the bacterium in manufacture of a composition for decomposing an opioid peptide or a drug for decomposing an opioid peptide.

[2] A *Bifidobacterium* bacterium, a culture of the bacterium, and/or a processed cell product of the bacterium, which is used for decomposing an opioid peptide.

[3] A *Bifidobacterium* bacterium, a culture of the bacterium, and/or a processed cell product of the bacterium, which is used for amelioration, prevention, or treatment of a disease that can be ameliorated, prevented, or treated by decomposition of an opioid peptide.

[4] Use of a *Bifidobacterium* bacterium, a culture of the bacterium, and/or a processed cell product of the bacterium for decomposing an opioid peptide.

[5] A method for decomposing an opioid peptide, which comprises the step of administering a *Bifidobacterium* bacterium, a culture of the bacterium, and/or a processed cell product of the bacterium to a mammal.

[6] A method for decomposing an opioid peptide, which comprises the step of administering an agent for decomposing an opioid peptide or composition for decomposing an opioid peptide containing a *Bifidobacterium* bacterium, a culture of the bacterium, and/or a processed cell product of the bacterium as an active ingredient to a mammal.

[7] A method for ameliorating, preventing, or treating a disease that can be ameliorated, prevented, or treated by decomposition of an opioid peptide, which comprises the step of administering a *Bifidobacterium* bacterium, a culture of the bacterium, and/or a processed cell product of the bacterium to a mammal.

[8] A method for ameliorating, preventing, or treating a disease that can be ameliorated, prevented, or treated by decomposition of an opioid peptide, which comprises the step of administering an agent for decomposing an opioid peptide or composition for decomposing an opioid peptide containing a *Bifidobacterium* bacterium, a culture of the bacterium, and/or a processed cell product of the bacterium as an active ingredient to a mammal.

Examples

Hereafter, the present invention will be explained more in detail with reference to examples. However, the present invention is not limited by these examples.

[Test Example 1]

A test confirming that *Bifidobacterium* bacteria have the DPP-4 activity was performed.

(1) Preparation of culture

A cell suspension (90 μL) of each of the following seventeen kinds of *Bifidobacterium* bacteria (twelve kinds of HRB and five kinds of non-HRB), which had been cryopreserved in an aqueous solution containing 1% sodium glutamate and 10% skim milk powder, was added to 3 mL of the MRS liquid medium, and anaerobic culture was carried out at 37°C for 16 hours so that the cell number of the *Bifidobacterium* bacterium in the culture became 1 x $10^9$ CFU/mL. The MRS liquid medium was prepared by dissolving 5.5 g of Difco Lactobacilli MRS Broth (produced by BD), and 50 mg of L-cysteine monohydrochloride monohydrate (produced by Wako Pure Chemical Industries) in pure water so as to obtain a volume of 100 mL, adjusting the solution to pH 6.5 with an HCl aqueous solution, and sterilizing the solution at 121°C for 15 minutes.

<Human-residential *Bifidobacterium* bacteria (HRB)>

- *Bifidobacterium longum* subsp. *longum* ATCC 15707
- *Bifidobacterium longum* subsp. *longum* ATCC BAA-999
- *Bifidobacterium longum* subsp. *infantis* ATCC 15697
- *Bifidobacterium longum* subsp. *infantis* BCCM LMG23728
- *Bifidobacterium breve* ATCC 15700
- *Bifidobacterium breve* FERM BP-11175
- *Bifidobacterium breve* BCCM LMG23729
- *Bifidobacterium bifidum* ATCC 29521
- *Bifidobacterium adolescentis* ATCC 15703
- *Bifidobacterium angulatum* ATCC 27535
- *Bifidobacterium dentium* DSM 20436
- *Bifidobacterium pseudocatenulatum* ATCC 27919

<Nonhuman-residential *Bifidobacterium* bacteria (non-HRB)>

- *Bifidobacterium animalis* subsp. *lactis* DSM 10140
- *Bifidobacterium animalis* subsp. *animalis* ATCC 25527
- *Bifidobacterium pseudolongum* subsp. *globosum* JCM 5820
- *Bifidobacterium pseudolongum* subsp. *pseudolongum* ATCC 25526
- *Bifidobacterium thermophilum* ATCC 25525

(2) Measurement of DPP-4 activity

Each culture prepared in (1) was centrifuged under the conditions of 4°C and 5000 x g for 30 minutes, then the supernatant was discarded, and the separated cells were suspended in a PBS solution. Each suspension was prepared at a turbidity (OD600) of 0.1, H-Gly-Pro-AMC·HBr (produced by BACHEM), which is a fluorescent substrate specific to DPP-4, was added, and anaerobic culture was performed at 37°C for 60 minutes. After completion of the culture, the fluorescence intensity of the culture was measured at an excitation wavelength of 360 nm and a measurement wavelength of 460 nm with Microplate Reader SH-9000 (produced by Corona Electric). The unit of the fluorescence intensity was arbitrary unit (a.u.). From the measured fluorescence intensity, the concentration (nM) of the fluorescent substance derived from the fluorescent substrate, aminomethylcoumarin (AMC), in the culture was calculated by using a calibration curve representing a relationship between the concentration of AMC and the fluorescence intensity at the measurement wavelength of 460 nm, which was created beforehand. Furthermore, the enzymatic activity of each *Bifidobacterium* bacterium was calculated from the AMC concentration in terms of the enzyme unit, with which the enzymatic activity for producing 1 nmol of AMC in 1 minute is represented as 1 unit (U).

(3) Results

The fluorescence intensities and the enzymatic activities of the *Bifidobacterium* bacteria were as shown in Tables 1-1 and 1-2. It was confirmed that all the seventeen kinds of the *Bifidobacterium* bacteria show an activity for decomposing a fluorescence substrate specific to DPP-4. That is, it was confirmed that all the seventeen kinds of the *Bifidobacterium* bacteria have the enzymatic activity of DPP-4. There was also confirmed a tendency that the DPP-4 activities of the *Bifidobacterium* bacteria as HRB were higher than those of the *Bifidobacterium* bacteria as non-HRB. Furthermore, the DPP-4 activities of the infant-residential *Bifidobacterium* bacteria, such as *Bifidobacterium longum* subsp. *infantis* ATCC 15697, *Bifidobacterium breve* FERM BP-11175, *Bifidobacterium breve* BCCM LMG23729, and *Bifidobacterium breve* ATCC 15700, were especially high among HRB, and they showed the enzymatic activities higher than the DPP-4 activity of *Bifidobacterium animalis* subsp. *animalis* ATCC 25527 as non-HRB by more than 5 times. Thus, it was suggested that they have strong DPP-4 activity.

[Table 1-1]

Table 1-1

|  | Fluorescence intensity (a.u.) | Enzymatic activity (mU) |
|---|---|---|
| Bifidobacterium longum subsp. longum ATCC15707 | 8999 | 0.87 |
| Bifidobacterium longum subsp. longum ATCC BAA-999 | 9322 | 0.90 |
| Bifidobacterium longum subsp. infantis ATCC15697 | 11219 | 1.09 |
| Bifidobacterium longum subsp. infantis BCCM LMG23728 | 7950 | 0.76 |
| Bifidobacterium breve ATCC15700 | 11529 | 1.12 |
| Bifidobacterium breve FERM BP-11175 | 14829 | 1.45 |
| Bifidobacterium breve BCCM LMG23729 | 11817 | 1.15 |
| Bifidobacterium bifidum ATCC29521 | 7926 | 0.76 |
| Bifidobacterium adolescentis ATCC15703 | 9316 | 0.90 |
| Bifidobacterium angulatum ATCC27535 | 3853 | 0.35 |
| Bifidobacterium dentium DSM20436 | 5850 | 0.55 |

[Table 1-2]

Table 1-2

|  | Fluorescence intensity (a.u.) | Enzymatic activity (mU) |
|---|---|---|
| Bifidobacterium psudocatenulatum ATCC27919 | 4553 | 0.42 |
| Bifidobacterium animalis subsp. lactis DSM10140 | 3984 | 0.37 |
| Bifidobacterium animalis subsp. animalis ATCC25527 | 2432 | 0.21 |
| Bifidobacterium pseudolongum subsp. globosum JCM5820 | 2989 | 0.27 |
| Bifidobacterium pseudolongum subsp. pseudolongm ATCC25526 | 4221 | 0.39 |
| Bifidobacterium thermophilum ATCC25525 | 7067 | 0.68 |

[Test Example 2]

Further, a test for identifying high DPP-4 activity fractions of *Bifidobacterium breve* FERM BP-11175 as HRB, and *Bifidobacterium animalis* subsp. *lactis* DSM10140 as non-HRB was performed.

(1) Preparation of culture

Cultures of the aforementioned two kinds of *Bifidobacterium* bacteria were prepared in the same manner as that of Test Example 1, (1).

(2) Disruption treatment by ultrasonication

Each culture prepared in (1) was centrifuged under the conditions of 4°C and 5000 x g for 30 minutes, then the supernatant was discarded, and the separated cells were suspended in a PBS solution to obtain "cell suspension". Each suspension was ultrasonicated by using BRANSON SONIFIER 250 (produced by Branson Ultrasonics) to disrupt the bacterial cells, and then centrifuged at 16000 x g and 4°C for 30 minutes. Then, the supernatant was collected as a "water-soluble fraction", and the precipitates were suspended again in a PBS solution to obtain "precipitate re-suspension".

(3) Measurement of DPP-4 activity

The enzymatic activities of the water-soluble fractions and precipitate re-suspensions collected in (2) were calculated by measuring fluorescence intensities according to the procedure used in Test Example 1, (2).

(4) Results

The enzymatic activities of the samples were as shown in Table 2. It was confirmed that the enzymatic activity of the water-soluble fraction was higher than the enzymatic activity of the precipitate re-suspension for both the *Bifidobacterium* bacteria. Furthermore, the enzymatic activity of the water-soluble fraction was higher than the enzymatic activity of the cell suspension confirmed in Test Example 1 for both the *Bifidobacterium* bacteria.

As for the water-soluble fractions of the aforementioned two kinds of strains, the activity of *Bifidobacterium breve* FERM BP-11175 as HRB was higher than the activity of *Bifidobacterium animalis* subsp. *lactis* DSM10140 as non-HRB, and the former was higher than the latter by more than 1.3 times. Therefore, it was suggested that water-soluble fractions have marked opioid peptide decomposition activity.

[Table 2]

Table 2

| | Enzymatic activity (mU) | |
|---|---|---|
| | Water-soluble fraction | Precipitate re-suspension |
| Bifidobacterium breve FERM BP-11175 | 6.4 | 1.4 |
| Bifidobacterium animalis subsp. lactis DSM10140 | 4.6 | 0.5 |

[Test Example 3]

Furthermore, a test was performed for confirming activity for decomposing β-casomorphin as an opioid peptide of the following four kinds of strains chosen from the *Bifidobacterium* bacteria confirmed to have the DPP-4 activity in Test Example 1. As for the two kinds of the strains, *Bifidobacterium breve* FERM BP-11175 and *Bifidobacterium animalis* subsp. *lactis* DSM10140, gliadorphin decomposition activity was also confirmed.

- *Bifidobacterium breve* BCCM LMG23729
- *Bifidobacterium breve* FERM BP-11175
- *Bifidobacterium animalis* subsp. *lactis* DSM10140
- *Bifidobacterium animalis* subsp. *animalis* ATCC 25527

(1) Preparation of culture

Cultures of the aforementioned four kinds of the *Bifidobacterium* bacteria were prepared in the same manner as that of Test Example 1, (1).

(2) Measurement of β-casomorphin and gliadorphin decomposition activities

Each culture prepared in (1) was centrifuged under the conditions of 4°C and 5000 x g for 30 minutes, then the supernatant was discarded, and the separated cells were suspended in a PBS solution. The cell density of each suspension was adjusted ($10^{11}$ cells/ml) by measuring cell number using a cell counter (DU-AA01 NP-H, produced by Panasonic), then β-casomorphin (β-Casomorphin 1-7 human, produced by SIGMA-ALDORICH) or gliadorphin (Gliadorphin-7, produced by Bachem) was added to the suspension at a concentration of 100 μg/ml, and anaerobic culture was performed at 37°C for 1 hour. After the culture, the suspension was centrifuged under the conditions of 4°C and 5000 x g for 30 minutes, and the supernatant was collected and analyzed by HPLC (D-7000 HPLC System, produced by Hitachi) under the following conditions to measure the concentration of remaining β-casomorphin or gliadorphin.

[HPLC conditions]

Column: POROS R2/H, 2.1 mmD/100 mmL (produced by PerSeptive Biosystems)
Detection: 280 nm
Flow rate: 0.4 ml/minute
Eluate A: Water containing 0.1% TFA
Eluate B: Acetonitrile containing 0.1% TFA
Gradient: Linear gradient in which ratio of the eluent B is increased from 5% to 60% in 40 minutes
Then, β-casomorphin consumption ratio (ratio of decomposed β-casomorphin) was obtained in accordance with the following equation.

```
[β-Casomorphin consumption ratio (%)] = 100 - [(β-Casomorphin
concentration in culture after cultivation)/(β-Casomorphin
concentration in culture before cultivation)] x 100
```

Gliadorphin consumption ratio (ratio of decomposed gliadorphin) was also obtained in accordance with the following equation.

```
[Gliadorphin consumption ratio (%)] = 100 - [(Gliadorphin
concentration in culture after cultivation)/(Gliadorphin
concentration in culture before cultivation)] x 100
```

(3) Results

The consumption ratios of β-casomorphin or gliadorphin (ratios of decomposed β-casomorphin or gliadorphin) for the *Bifidobacterium* bacteria were as shown in Table 3.

[Table 3]

Table 3

| | β-Casomorphin consumption ratio | Gliadorphin consumption ratio |
|---|---|---|
| Bifidobacterium breve BCCM LMG23729 | 93% | - |
| Bifidobacterium breve FERM BP-11175 | 76% | 10% |
| Bifidobacterium animalis subsp. lactis DSM10140 | 59% | 6% |
| Bifidobacterium animalis subsp. animalis ATCC25527 | 5% | - |

It was confirmed that all the *Bifidobacterium* bacteria decompose β-casomorphin. *Bifidobacterium breve* BCCM LMG23729 and *Bifidobacterium breve* FERM BP-11175, which are HRB, showed high β-casomorphin consumption ratios as high as 93% and 76%, respectively. On the other hand, *Bifidobacterium animalis* subsp. *lactis* DSM10140 and *Bifidobacterium animalis* subsp. *animalis* ATCC 25527, which are non-HRB, showed β-casomorphin consumption ratios of 59% and 5%, respectively.

It was also confirmed that all the *Bifidobacterium* bacteria decompose gliadorphin. The gliadorphin consumption ratio of *Bifidobacterium breve* FERM BP-11175 which is HRB, was 10%, whereas the gliadorphin consumption ratio of *Bifidobacterium animalis* subsp. *lactis* DSM10140, which is non-HRB, was 6%.

Therefore, it was suggested that the *Bifidobacterium* bacteria as HRB can more effectively decompose the opioid peptides, β-casomorphin and gliadorphin, compared with non-HRB. Furthermore, both *Bifidobacterium breve* FERM BP-11175 and *Bifidobacterium animalis* subsp. *lactis* DSM10140 showed β-casomorphin consumption ratios higher than the gliadorphin consumption ratios by as much as more than 7 times. Therefore, it was suggested that the *Bifidobacterium* bacteria used in the present invention have high decomposition activity especially for β-casomorphin as opioid peptide.

[Test Example 4]

Furthermore, a test was performed for confirming whether substrate specificities for β-casomorphin of *Bifidobacterium breve* BCCM LMG23729, which is HRB, and *Bifidobacterium animalis* subsp. *animalis* ATCC 25527, which is non-HRB, are different by using water-soluble fractions of the *Bifidobacterium* bacteria, which show the highest DPP-4 activity.

(1) Preparation of cultures

Cultures of the aforementioned two kinds of *Bifidobacterium* bacteria were prepared in the same manner as that of Test Example 1, (1), and water-soluble fractions were collected by the procedure of Test Example 2, (2). When the DPP-4 activity of the water-soluble fraction of *Bifidobacterium animalis* subsp. *animalis* ATCC 25527 was confirmed in the same manner as that of Test Example 2, it showed a fluorescence intensity corresponding to 0.4 time of that provided by the water-soluble fraction of *Bifidobacterium breve* BCCM LMG23729, which is HRB. Therefore, the water-soluble fraction of *Bifidobacterium breve* BCCM LMG23729, which is HRB, was diluted 2.5 times, so that the same volumes of the water-soluble fractions of the bacteria should show the same DPP-4 activity.

(2) Measurement of β-casomorphin decomposition activity

To each of the 2.5-fold dilution of the water-soluble fraction of *Bifidobacterium breve* BCCM LMG23729 as HRB, and the water-soluble fraction of *Bifidobacterium animalis* subsp. *animalis* ATCC 25527 as non-HRB, which were prepared in (1) described above, β-casomorphin (β-Casomorphin 1-7 human, produced by SIGMA-ALDORICH) was added at a concentration of 100 μg/ml, and anaerobic culture was performed at 37°C for 1 hour. After the culture, each culture was centrifuged under the conditions of 4°C and 5000 x g for 30 minutes, the supernatant was collected, and the β-casomorphin consumption ratio (ratio of decomposed β-casomorphin) was obtained by the same procedure as that of Test Example 3, (2).

(3) Results

The β-casomorphin consumption ratios (ratios of decomposed β-casomorphin) of the *Bifidobacterium* bacteria were as shown in Table 4.

[Table 4]

Table 4

|  | β-Casomorphin consumption ratio |
|---|---|
| Bifidobacterium breve BCCM LMG23729 | 45% |
| Bifidobacterium animalis subsp. animalis ATCC25527 | 31% |

*Bifidobacterium breve* BCCM LMG23729, which is HRB, and *Bifidobacterium animalis* subsp. *animalis* ATCC 25527, which is non-HRB, showed a difference of more than 10% in the β-casomorphin (opioid peptide) consumption ratio (ratio of decomposed β-casomorphin) even in the comparison using samples of which concentrations were adjusted so that DPP-4 activities should be the same. It was estimated that this result was provided by a higher substrate specificity of *Bifidobacterium breve* BCCM LMG23729 as HRB for β-casomorphin as opioid peptide, compared with that of *Bifidobacterium animalis* subsp. *animalis* ATCC 25527 as non-HRB. Therefore, this result also suggested that *Bifidobacterium* bacteria as HRB can more effectively decompose β-casomorphin, which is an opioid peptide, compared with non-HRB.

[Preparation Example 1]

One or more kinds of bacteria selected from the seventeen kinds of the *Bifidobacterium* bacteria used in Test Example 1 are added to 3 mL of the MRS liquid medium for each strain or the same MRS liquid medium, anaerobic culture is carried out at 37°C for 16 hours, and the culture is concentrated and lyophilized to obtain cell powder of the one or more kinds of bacteria. The cell powder of the one or more kinds of bacteria is appropriately mixed with an excipient etc., and the mixture is made into tablets. The tablets are taken every day for three months so that the total intake of the bacteria is $1 \times 10^6$ to $1 \times 10^{12}$ cfu/kg of body weight/day.
Intake of the tablets is expected to provide an opioid peptide decomposition effect.

[Preparation Example 2]

One or more kinds of bacteria selected from the seventeen kinds of the *Bifidobacterium* bacteria used in Test Example 1 are added to 3 mL of the MRS liquid medium for each strain or the same MRS liquid medium, anaerobic culture is carried out at 37°C for 16 hours, and the culture is concentrated and lyophilized to obtain cell powder of the one or more kinds of bacteria. The cell powder of the one or more kinds of bacteria is added to a fermented milk material to obtain fermented milk. The fermented milk is taken every day for at least three months so that the total intake of the bacteria is $1 \times 10^6$ to $1 \times 10^{12}$ cfu/kg of body weight/day.
Intake of the fermented milk is expected to provide an opioid peptide decomposition effect.

[Preparation Example 3]

A method for producing a powdered infant formula using one or more kinds of bacteria selected from the seventeen kinds of the *Bifidobacterium* bacteria used in Test Example 1 is shown below.
Desalted milk serum protein powder (10 kg, produced by Milei), milk casein powder (6 kg, produced by Fonterra), lactose (48 kg, produced by Milei), mineral mixture (920 g, produced by Tomita Pharmaceutical), vitamin mixture (32 g, produced by Tanabe Seiyaku), lactulose (500 g, produced by Morinaga Milk Industry), raffinose (500 g, produced by Nippon Beet Sugar Mfg.), and galactooligosaccharide liquid sugar (900 g, produced by Yakult Pharmaceutical Industry) are dissolved in warm water (300 kg), and further dissolved by heating at 90°C for 10 minutes, modified fat (28 kg, produced by TAIYO YUSHI) is added, and the mixture is homogenized. Then, the mixture is sterilized, concentrated, and spray-dried to prepare powdered infant formula (about 95 kg). Cell powder obtained by adding one or more kinds of bacteria selected from the seventeen kinds of the *Bifidobacterium* bacteria used in Test Example 1 to 3 mL of the MRS liquid medium for each strain or the same MRS liquid medium, carrying out anaerobic culture at 37°C for 16 hours, concentrating and lyophilizing the culture, and triturating the obtained cells with starch (100 g, $1.8 \times 10^{11}$ cfu/g) was added to the formula obtained above to prepare about 95 kg of a bifidobacterium and oligosaccharide-formulated powdered infant formula. If the obtained powdered infant formula is dissolved in water to prepare a liquid infant formula having a total solid concentration of 14% (w/v), which is a normal concentration for milk preparation, the number of the bifidobacteria in the liquid formula is $2.7 \times 10^9$ cfu/100 ml. Intake of the powdered infant formula obtained as described above is expected to provide an opioid peptide decomposition effect.

**Claims**

1. A composition for decomposing an opioid peptide containing a *Bifidobacterium* bacterium, a culture of the bacterium, and/or a processed cell product of the bacterium as an active ingredient.

2. The composition for decomposing an opioid peptide according to claim 1, wherein the opioid peptide is β-casomorphin.

3. The composition for decomposing an opioid peptide according to claim 1 or 2, wherein the processed cell product of the *Bifidobacterium* bacterium is a water-soluble fraction of a disrupted cell product of the bacterium.

4. The composition for decomposing an opioid peptide according to any one of claim 1 to 3, wherein the *Bifidobacterium* bacterium is an infant-residential bacterium.

5. The composition for decomposing an opioid peptide according to any one of claim 1 to 4, wherein the *Bifidobacterium* bacterium is one or more selected from the group consisting of *Bifidobacterium longum* subsp. *longum* ATCC 15707, *Bifidobacterium longum* subsp. *longum* ATCC BAA-999, *Bifidobacterium longum* subsp. *infantis* ATCC 15697, *Bifidobacterium longum* subsp. *infantis* BCCM LMG23728, *Bifidobacterium breve* ATCC 15700, *Bifidobacterium breve* FERM BP-11175, *Bifidobacterium breve* BCCM LMG23729, *Bifidobacterium bifidum* ATCC 29521, *Bifidobacterium adolescentis* ATCC 15703, *Bifidobacterium angulatum* ATCC 27535, *Bifidobacterium dentium* DSM20436, *Bifidobacterium pseudocatenulatum* ATCC 27919, *Bifidobacterium animalis* subsp. *lactis* DSM10140, *Bifidobacterium animalis* subsp. *animalis* ATCC 25527, *Bifidobacterium pseudolongum* subsp. *globosum* JCM5820, *Bifidobacterium pseudolongum* subsp. *pseudolongum* ATCC 25526, and *Bifidobacterium thermophilum* ATCC 25525.

6. The composition for decomposing an opioid peptide according to any one of claim 1 to 4, wherein the *Bifidobacterium* bacterium is one or more selected from the group consisting of *Bifidobacterium longum* subsp. *longum* BB536 (NITE BP-02621), *Bifidobacterium longum* subsp. *infantis* M-63 (NITE BP-02623), and *Bifidobacterium breve* M-16V (NITE BP-02622).

7. A pharmaceutical composition for decomposing an opioid peptide containing a *Bifidobacterium* bacterium, a culture of the bacterium, and/or a processed cell product of the bacterium as an active ingredient.

8. The pharmaceutical composition according to claim 7, which is used for prevention and/or treatment of autism, Asperger's syndrome, Rett's disorder, childhood disintegrative disorder, or sleep apnea.

9. A food or drink composition for decomposing an opioid peptide containing a *Bifidobacterium* bacterium, a culture of the bacterium, and/or a processed cell product of the bacterium as an active ingredient.

10. Use of a *Bifidobacterium* bacterium, a culture of the bacterium, and/or a processed cell product of the bacterium in manufacture of a composition for decomposing an opioid peptide.

11. A *Bifidobacterium* bacterium, a culture of the bacterium, and/or a processed cell product of the bacterium, which is used for decomposing an opioid peptide.

12. Use of a *Bifidobacterium* bacterium, a culture of the bacterium, and/or a processed cell product of the bacterium for decomposing an opioid peptide.

13. A method for decomposing an opioid peptide, which comprises the step of administering a *Bifidobacterium* bacterium, a culture of the bacterium, and/or a processed cell product of the bacterium to a mammal.

## INTERNATIONAL SEARCH REPORT

| | International application No. |
|---|---|
| | PCT/JP2018/011920 |

### A. CLASSIFICATION OF SUBJECT MATTER
Int. Cl. A61K35/745(2015.01)i, A23L33/135(2016.01)i, A61P25/00(2006.01)i, A61P43/00(2006.01)i, C12N1/00(2006.01)i, C12N1/20(2006.01)i

According to International Patent Classification (IPC) or to both national classification and IPC

### B. FIELDS SEARCHED

Minimum documentation searched (classification system followed by classification symbols)
Int. Cl. A61K35/745, A23L33/135, A61P25/00, A61P43/00, C12N1/00, C12N1/20

Documentation searched other than minimum documentation to the extent that such documents are included in the fields searched
```
Published examined utility model applications of Japan     1922-1996
Published unexamined utility model applications of Japan   1971-2018
Registered utility model specifications of Japan           1996-2018
Published registered utility model applications of Japan   1994-2018
```

Electronic data base consulted during the international search (name of data base and, where practicable, search terms used)
JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X<br>Y | ISHIZEKI, S. et al., Effect of administration of bifidobacteria on intestinal microbiota in low-birth-weight infants and transition of administered bifidobacteria: A comparison between one-species and three-species administration, Anaerobe, 2013, vol. 23, pp. 38-44, abstract | 11<br>5-6 |
| X<br>Y | CN 105213433 A (WANG, Yichao) 06 January 2016, claims 1-8, examples 1-4, paragraphs [0072]-[0087], 「自閉症の臨床観察」 (Clinical Observations of Autism) (Family: none) | 1-4, 7-13<br>5-6 |
| A | CN 104011199 A (MORINAGA MILK INDUSTRY CO., LTD.) 27 August 2014, claim 1 & US 2015/0139955 A1, claim 1 & WO 2013/094250 A1 & EP 2796542 A1 | 1-13 |

☒ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06.06.2018 | 19.06.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

# EP 3 603 655 A1

## INTERNATIONAL SEARCH REPORT

International application No.

PCT/JP2018/011920

C (Continuation). DOCUMENTS CONSIDERED TO BE RELEVANT

| Category* | Citation of document, with indication, where appropriate, of the relevant passages | Relevant to claim No. |
|---|---|---|
| Y | WALLACE, R. J. et al., Peptidase activity of human colonic bacteria, Anaerobe, 1997, vol. 3, no. 4, pp. 251-257, table 3, p. 254, left column, 5$^{th}$ to 15$^{th}$ lines from the bottom | 1-13 |
| Y | PUSCHEL, G. et al., Isolation and characterization of dipeptidyl peptidase IV from human placenta, Eur. J. Biochem., 1982, vol. 126, no. 2, pp. 359-365, p. 359, entire text, left column, lines 7-13, table 5 | 1-6, 10-12 |
| Y | WO 2003/028745 A1 (NEUROZYM BIOTECH AS) 10 April 2003, claims 27-33, tables III-V, page 4, lines 3-8 & US 2004/0247581 A1 & EP 1448211 A1 & NO 20014788 A & AT 400282 T | 1-13 |
| A | WO 2013/047082 A1 (AMANO ENZYME INC.) 04 April 2013 & US 2014/0219982 A1 & EP 2762151 A1 | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013047082 A
- JP 2011507540 A
- JP 3050074 B
- JP 2920818 B

### Non-patent literature cited in the description

- **M.S. TRIVEDI et al.** *J. Nutr. Biochem.,* 2014, vol. 25 (10), 1011-1018
- **A. REISSMANN et al.** *Functional Foods in Health and Disease,* 2014, vol. 4 (8), 349-361
- **T-R YAN et al.** *Biosci. Biotech. Biochem.,* 1992, vol. 56 (5), 704-707
- **G. PUSCHEL et al.** *Eur. J. Biochem.,* 1982, vol. 126 (2), 359-365
- **O. SOKOLOV et al.** *Peptides,* 2014, vol. 56, 68-71
- **J. WASILEWSKA et al.** *Nuropeptides,* 2011, vol. 45, 189-195